(19) Europäisches Patentamt
European Patent Office
Office européen des brevets

(11) **EP 3 225 996 B1**

(12) **EUROPEAN PATENT SPECIFICATION**

(45) Date of publication and mention of the grant of the patent:
**10.07.2019 Bulletin 2019/28**

(51) Int Cl.:
***G01N 33/543*** *(2006.01)*  ***G01N 33/68*** *(2006.01)*
***G01N 33/92*** *(2006.01)*

(21) Application number: **17164039.4**

(22) Date of filing: **31.03.2017**

(54) **METHOD FOR ASSISTING DIAGNOSIS OF RISK OF PROGRESSION TO NEPHROPATHY AND USE OF REAGENT KIT**

VERFAHREN ZUR UNTERSTÜTZUNG DER DIAGNOSE DES RISIKOS EINES FORTSCHREITENS VON NEPHROPATHIE UND VERWENDUNG EINES REAGENZIENKITS

PROCÉDÉ POUR FACILITER LE DIAGNOSTIC DU RISQUE DE PROGRESSION VERS UNE NÉPHROPATHIE ET UTILISATION D'UN KIT DE RÉACTIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2016 JP 2016071819 P**

(43) Date of publication of application:
**04.10.2017 Bulletin 2017/40**

(73) Proprietor: **SYSMEX CORPORATION**
**Kobe-shi**
**Hyogo 651-0073 (JP)**

(72) Inventors:
• **SHIRAHASE, Yasushi**
  **Hyogo 651-0073 (JP)**
• **YOSHIDA, Toshiyuki**
  **Hyogo 651-0073 (JP)**
• **HOTTA, Osamu**
  **Hyogo 651-0073 (JP)**

(74) Representative: **De Clercq & Partners**
**Edgard Gevaertdreef 10a**
**9830 Sint-Martens-Latem (BE)**

(56) References cited:
**EP-A1- 1 300 683    JP-A- 2004 069 672**

• **CALANDRA S ET AL: "Plasma and urine lipoproteins during the development of nephrotic syndrome induced in the rat by adriamycin", EXPERIMENTAL AND MOLECULAR PATHOLOGY, ACADEMIC PRESS, US, vol. 39, no. 3, 1 December 1983 (1983-12-01), pages 282-299, XP026216373, ISSN: 0014-4800, DOI: 10.1016/0014-4800(83)90058-8 [retrieved on 1983-12-01]**
• **Gomo Zvenyika ET AL: "High-Density Lipoprotein Apolipoproteins in Urine: II. Enzyme-Linked Immunoassay of Apolipoprotein A-I", CLIN. CHEM. 34/9, 1 January 1988 (1988-01-01), pages 1781-1786, XP055006657, Retrieved from the Internet: URL:http://www.clinchem.org/cgi/reprint/34/9/1781.pdf [retrieved on 2011-09-09]**
• **BRITES ET AL: "Chronic renal failure in diabetic patients increases lipid risk factors for atherosclerosis", DIABETES RESEARCH AND CLINICAL PRAC, AMSTERDAM, NL, vol. 75, no. 1, 8 December 2006 (2006-12-08), pages 35-41, XP005798082, ISSN: 0168-8227, DOI: 10.1016/J.DIABRES.2006.05.011**

**Description**

TECHNICAL FIELD

The present invention relates to a method for assisting a diagnosis of a risk of progression to nephropathy in a diabetes patient who is at pre-nephropathy. The present invention also relates to a use of a reagent kit for a diagnosis of a risk of progression to nephropathy.

BACKGROUND

Diabetic nephropathy is one of the complications of diabetes. Diabetic nephropathy is a renal disease which progresses through a plurality of stages. Disease stages of diabetic nephropathy are classified into five stages from pre-nephropathy (Stage 1) to dialysis therapy (Stage 5). At the stages of pre-nephropathy (Stage 1) and incipient nephropathy (Stage 2), a patient has only a limited number of subjective symptoms. However, nephropathy may progress to failure in due course, and a dialysis treatment will be required. The dialysis treatment is a large burden on a patient. In recent years, the number of the patients who require the dialysis treatment is increasing, and medical expenses are also increasing in association with such increase in the number of patients.

In the case of diabetic nephropathy, kidney functions can be restored if an appropriate treatment can be started at an early stage. However, at a more advanced stage, it is difficult to maintain and to restore kidney functions by a treatment, though the progression of the disease can be delayed. Accordingly, conducting a treatment early by finding a patient who is at a risk of progression to diabetic nephropathy at an early stage where the restoration of kidney functions can be expected reduces the future burden on the patient. Such a treatment also contributes to a reduction in medical expenses to a large extent.

At present, measurements of a glomerular filtration rate (GFR), a value of urine albumin and the like are conducted, for testing diabetic nephropathy. Various biomarkers are also utilized for the diagnosis of nephropathy. For example, United States Patent Application Publication No. 2003/0017523 describes that renal failure is examined based on apolipoprotein A1 (ApoA1) in urine. Gomo ZA., et al., High-Density Lipoprotein Apolipoproteins in Urine: II. Enzyme-Linked Immunoassay of Apolipoprotein A-I, Clinical Chemistry, Vol. 34, No. 9, 1781 - 1786 (1988) describe that there was a difference of thousandfold or more in the concentration of ApoA1 in urine between healthy subjects and patients with renal dysfunction. Based on this result, Gomo ZA., et al., High-Density Lipoprotein Apolipoproteins in Urine: II. Enzyme-Linked Immunoassay of Apolipoprotein A-I, Clinical Chemistry, Vol. 34, No. 9, 1781 - 1786 (1988) describe a perspective that the measurement of ApoA1 in urine makes it possible that healthy subjects and patients with asymptomatic nephropathy are discriminated from each other. Patel ML. et al., Clinical Correlation between ApoB/Apo-A1 Ratio in Type 2 Diabetic Nephropathy-A Tertiary Centre Experience, Int. J. Sci. Res. Pub, Vol. 2, Issue 9, 1 - 8 (2012) suggest the possibility that the ratio of the concentration of apolipoprotein B (ApoB) and that of ApoA1 in blood serum (serum ApoB/ApoA1 ratio) may be an early indicator of diabetic nephropathy. Calandra, et al, Experimental and Molecular Pathology, 39, 282-299 (1983), discloses that apolipoprotein A1 is increased in nephrotic syndrome form mild to severe in rats treated with adriamycin. Brites, et al, Diabetes Research and Clinical Practice, 75, 35-41, 2007, discloses that diabetic patients with glomerulopathy and chronic renal failure showed detectable levels of Apo-A1. Publication EP1300683A1, discloses methods for examining nephropathy wherein apopoliprotein A1 is measured in a urine sample from patients.

Gomo ZA., et al, High-Density Lipoprotein Apolipoproteins in Urine: II. Enzyme-Linked Immunoassay of Apolipoprotein A-I, Clinical Chemistry, Vol. 34, No. 9, 1781 - 1786 (1988) describe that whether a subject is healthy or is affected with renal dysfunction at a given moment can be determined by the concentration of ApoA1 in urine. However, this document does not describe or suggest diagnosis of a risk of progression, i.e. determining whether a diabetes patient who is at pre-nephropathy will progress to more advanced nephropathy in the future based on the concentration of ApoA1 in urine. As described above, finding a diabetes patient who is at a risk of progression of nephropathy at an early stage is important to start treatment. Accordingly, the development of a means which enables a diagnosis of such a risk is desired.

SUMMARY OF THE INVENTION

The present invention provides a method for assisting a diagnosis of a risk of progression to nephropathy, the method including the steps of: measuring a concentration of ApoA1 in urine of a diabetes patient who is at pre-nephropathy; and determining that the patient is at a high risk of progression to nephropathy when a value of the concentration of ApoA1 in urine is equal to or higher than a predetermined threshold value or determining that the patient is at a low risk of progression to nephropathy when the value of the concentration of ApoA1 in urine is lower than the predetermined threshold value. Accordingly, the invention provides a method for assisting a diagnosis of a risk of progression or the prognosis of a patient who is at pre-nephropathy for developing nephropathy.

In one embodiment of the method, the concentration of ApoA1 in urine is measured by using a capturing body which specifically binds to ApoA1.

In one embodiment of the method, the capturing body which specifically binds to ApoA1 is an anti-ApoAI antibody.

In one embodiment of the method, the diabetes patient who is at pre-nephropathy has a urine albumin-to-creatinine ratio of lower than 30 mg/gCr and has an estimated GFR of equal to or higher than 30 mL/min/1.73 m$^2$.

In one embodiment of the method, the value of the concentration of ApoA1 in urine is a value corrected by a value of a concentration of creatinine in urine of the patient.

In one embodiment of the method, the concentration of ApoA1 is measured by immunoassay in the measuring step.

The present invention provides a use of a reagent kit for the above-stated method. The reagent kit comprises: a first capturing body which specifically binds to ApoA1 for capturing ApoA1; a second capturing body which specifically binds to ApoA1 for detecting ApoA1; and a label substance.

In one embodiment of the use, the first capturing body is an anti-ApoAI antibody.

In one embodiment of the use, the second capturing body is an anti-ApoAI antibody.

In one embodiment of the use, the label substance is at least one selected from the group consisting of fluorescent substance, radioisotope and enzyme.

In one embodiment of the use, the reagent kit further comprises a solid phase, and the first capturing body is fixed on the solid phase.

In one embodiment of the use, the solid phase is a particle, membrane, microplate, microtube, or test tube.

In one embodiment of the use, the first capturing body is an anti-ApoAI antibody and the second capturing body is an anti-ApoAI antibody, and an epitope of the first capturing body is different from an epitope of the second capturing body.

According to the present invention, provided is determining a risk of progression to nephropathy in a diabetes patient who is at pre-nephropathy.

BRIEF DESCRIPTION OF THE DRAWINGS

Fig. 1 is a graph which shows the concentrations of ApoA1 and ApoB in samples (urine) of a progression group and a non-progression group of diabetic nephropathy 6 months after sampling in diabetes patients who were at pre-nephropathy.

Fig. 2 is a graph which shows the concentrations of ApoA1 and ApoB in samples (serum) of a progression group and a non-progression group of diabetic nephropathy 6 months after sampling in diabetes patients who were at pre-nephropathy.

Fig. 3A is an ROC (Receiver Operating Characteristic) curve which shows the accuracy of determination of a case where the risk of progression of nephropathy was determined based on the concentration of ApoA1 in urine of diabetes patients who were at pre-nephropathy.

Fig. 3B is an ROC curve which shows the accuracy of determination of a case where the risk of progression of nephropathy was determined based on the concentration of ApoA1 in urine of diabetes patients who were at incipient nephropathy.

Fig. 3C is an ROC curve which shows the accuracy of determination of a case where the risk of progression of nephropathy was determined based on the concentration of ApoA1 in urine of diabetes patients who were at overt nephropathy.

Fig. 4 is a graph which shows the concentrations of ApoA1 and ApoB in samples (urine) of a progression group and a non-progression group of diabetic nephropathy 6 months after sampling in diabetes patients who were at pre-nephropathy, incipient nephropathy and overt nephropathy.

Fig. 5A is a drawing which shows an example of the appearance of the reagent kit according to the present embodiment.

Fig. 5B is a drawing which shows an example of the appearance of the reagent kit according to the present embodiment.

Fig. 5C is a drawing which shows an example of the appearance of the reagent kit according to the present embodiment.

Fig. 5D is a drawing which shows an example of the appearance of the reagent kit according to the present embodiment.

Fig. 5E is a drawing which shows an example of the appearance of the reagent kit according to the present embodiment.

Fig. 5F is a drawing which shows an example of the appearance of the reagent kit according to the present embodiment.

DETAILED DESCRIPTION OF THE PREFERRED EMBODIMENTS

In the method for diagnosing the risk of progression from pre-nephropathy to nephropathy according to the present embodiment (hereinafter also referred to as "the diagnosis method"), firstly, a concentration of apolipoprotein A1 (ApoA1) in urine of a diabetes patient who is at pre-nephropathy is measured.

In the diagnosis method according to the present embodiment, a target of diagnosis is a diabetes patient who is at pre-nephropathy (hereinafter also referred to as "the subject"). Pre-nephropathy is one of disease stages of diabetic nephropathy. Pre-nephropathy is also called Stage 1. At present, with regard to the classification of the disease stages of diabetic nephropathy, reference is made to the classification of severities of chronic kidney disease (CKD) which is as shown in Table 1 below.

[Table 1]

| | | Albuminuria categories | | A1 | A2 | A3 |
|---|---|---|---|---|---|---|
| | | Urine albumin quantitation | | Normoalbuminuria | Microalbuminuria | Macroalbuminuria |
| | | Urine albumin-to-Cr ratio | | < 30 mg/gCr | 30 - 299 mg/gCr | > 300 mg/gCr |
| GFR categories (mL/min/ 1.73 m$^2$) | G1 | Normal or high | $\geq$ 90 | Pre-nephropathy (Stage 1) | Incipient nephropathy (Stage 2) | Overt nephropathy (Stage 3) |
| | G2 | Mildly decreased | 60 - 89 | | | |
| | G3a | Mildly to moderately decreased | 45 - 59 | | | |
| | G3b | Moderately to severely decreased | 30 - 44 | | | |
| | G4 | Severely decreased | 15 - 29 | Kidney failure (Stage 4) | | |
| | G5 | Kidney failure | < 15 | Dialysis therapy (Stage 5) | | |

In the present description, the terms "pre-nephropathy" and "Stage 1" refer to a disease stage in which the albuminuria category is A1 and the GFR category is G1, G2, G3a or G3b according to the classification of severities of CKD described in KDIGO (Kidney Disease: Improving Global Outcomes) 2012 Clinical Practice Guideline for the Evaluation and Management of Chronic Kidney Disease (see Table 1). In particular embodiments, the phrase "a diabetes patient who is at pre-nephropathy" refers to a patient who is diagnosed with diabetes, more particularly diagnosed as being at stage 1 pre-nephropathy. In particular embodiments, this may be a patient who has a urine albumin-to-creatinine (Cr) ratio of lower than 30 mg/gCr and an estimated GFR of equal to or higher than 30 mL/min/1.73 m$^2$. The method for diagnosing diabetes is not particularly limited, and the diagnosis may be conducted according to a known method. The methods for measuring urine albumin and creatinine as well as the method for calculating an estimated GFR are known in the art.

In the methods provided herein, urine collected from a subject is used as a sample. The urine collected from the subject is not particularly limited by the physical condition, the diet, the presence or absence of the medication of the subject, and the like. The timing of collecting urine is also not particularly limited. Any of urine in the early morning, spot urine and pooled urine may be used. In the method according to the present invention, a urine volume of about 100 to about 500 $\mu$L is sufficient.

As needed, a pretreatment may be performed on the urine collected from a subject such that the urine is suitable for a measurement of a concentration of ApoA1. Examples of such a pretreatment include dilution, adjustment of pH, and removal of insoluble solid components. For example, the dilution and the adjustment of pH of urine can be performed by mixing the urine with a buffer solution having a predetermined pH. The removal of insoluble solid components from urine can be performed by centrifugal separation. The buffer solution is not particularly limited as long as the buffer solution has a buffering action in a range of pH suitable for a measurement of a concentration of ApoA1. Examples of the buffer solution include Good's buffers such as Tris, MES, PIPES, TES and HEPES, and phosphate-buffered saline (PBS). When non-crystalline salts are present in urine, a chelating agent such as an EDTA salt may be added to urine in order to remove the non-crystalline salts. Hereinafter, urine collected from a subject and urine having been subjected to a pretreatment are both referred to as "the urine sample."

ApoA1 is known to be present in blood as a component of high-density lipoproteins (HDLs). ApoA1 is also present in urine by being excreted due to abnormality of kidney functions. ApoA1 may sometimes be present in urine in the form of being incorporated into a phagocyte such as a macrophage. Alternatively, it is also reported that the macrophage itself produces apoproteins. In particular embodiments, as a pretreatment of the urine, ApoA1 may be made to bereleased into the urine from phagocytes which incorporate ApoA1. The method for releasing ApoA1 from a cell is not particularly limited. Examples of the method include solubilization by using a detergent, and ultrasonic crushing. The solubilization of cells by using a detergent can be performed, for example, by mixing urine collected from a subject with a buffer solution containing a detergent. The amount of the buffer solution containing the detergent to be added can be appropriately set. The amount to be added is, for example, equal to or more than one-fold and/or equal to or less than 50-fold of the urine volume. Preferably, the buffer solution containing the detergent may be added in an amount equal to or more than one-fold and/or equal to or less than 10-fold of the urine volume. The kind of detergent is not particularly limited as long as the detergent does not interfere with the measurement of ApoA1. Examples of the detergent include Triton X-100, Tween 20, and octyl glucoside. The final concentration of the detergent in a urine sample can be appropriately set depending on the kind ofdetergent. For example, when Triton X-100 is used as a detergent, the final concentration of Triton X-100 in a urine sample is equal to or higher than 0.001% (v/v) and equal to or lower than 10% (v/v). Preferably, the final concentration of Triton X-100 in a urine sample is equal to or higher than 0.01% (v/v) and equal to or lower than 1% (v/v).

The concentration of ApoA1 in urine can be measured by a method which is capable of obtaining a value reflecting the concentration or amount of ApoA1 contained in a urine sample (hereinafter also referred to as "the measured value of ApoA1"). As such a method, a method by which ApoA1 is captured by using a capturing body which specifically binds to ApoA1 is preferable. The measured value of ApoA1 can be obtained by detecting ApoA1 captured by the capturing body according to a known method. Examples of the capturing body which specifically binds to ApoA1 include an antibody and an aptamer. Among them, an antibody is particularly preferable. The antibody may be any of a monoclonal antibody, a polyclonal antibody and fragments thereof (for example, Fab, and F(ab')2). The antibody which specifically binds to ApoA1 (hereinafter also referred to as "the anti-ApoAl antibody") itself is known in the art. The anti-ApoAl antibody is generally available.

In the methods provided herein, it is preferable to measure the concentration of ApoA1 in urine by an immunological measurement method by using the anti-ApoAl antibody. Examples of the immunological measurement method include an enzyme-linked immunosorbent assay (ELISA) method, an immune complex metastasis measurement method (see European Patent Application Publication No. 0,303,229 of which one of priority applications is Japanese Patent Application No. Sho 63-82350 corresponding to Japanese Patent Application Publication No. Hei 1-254868), an immunoturbidimetric method, an immunochromatographic method, and a latex agglutination method. A commercially available measurement kit such as an ELISA kit may be used. As an example of the measuring step, the case where the concentration of ApoA1 in urine is measured by a sandwich ELISA method is described below.

Firstly, a complex containing ApoA1 in urine, an antibody for capturing ApoA1 (hereinafter also referred to as "the capturing antibody") and an antibody for detecting ApoA1 (hereinafter also referred to as "the detecting antibody") is formed on a solid phase. The complex can be formed by mixing a urine sample, a capturing antibody and a detecting antibody. Then, a solution containing the complex is brought into contact with the solid phase which is capable of capturing the capturing antibody such that the complex can be formed on the solid phase. Alternatively, a solid phase on which a capturing antibody has been fixed beforehand may be used. In other words, the complex can be formed on the solid phase by contacting the solid phase on which the capturing antibody is fixed, the urine sample and the detecting antibody together. In the case where both the capturing antibody and the detecting antibody are monoclonal antibodies, it is preferable that the epitope for the capturing antibody and that for the detecting antibody are different from each other.

The way of fixing the capturing antibody on the solid phase is not particularly limited. For example, the capturing antibody and the solid phase may be bound directly or the capturing antibody and the solid phase may be bound indirectly through another substance. Examples of the direct binding include physical adsorption. Examples of the indirect binding include binding via a combination of biotin and avidin or streptavidin (hereinafter also referred to as "avidins"). In the latter case, the capturing antibody and the solid phase can be bound indirectly via binding of biotin and avidins by modifying the capturing antibody with biotin beforehand and binding avidins to the solid phase beforehand.

The material of the solid phase is not particularly limited. For example, the material of the solid phase can be selected from an organic high-molecular compound, an inorganic compound, a biopolymer and the like. Examples of the organic high-molecular compound include latex, polystyrene, and polypropylene. Examples of the inorganic compound include magnetic substances (iron oxide, chromium oxide, ferrite and the like), silica, alumina, and glass. Examples of the biopolymer include insoluble agarose, insoluble dextran, gelatin, and cellulose. Two or more kinds of them may be used in combination. The form of the solid phase is not particularly limited. Examples of the form of the solid phase include particles, a membrane, a microplate, a microtube, and a test tube.

The measured value of ApoA1 in urine can be obtained by detecting the complex formed on the solid phase according to a known method in the art. For example, when an antibody labeled with a label substance is used as the detecting antibody, the measured value of ApoA1 can be obtained by detecting a signal generated by the label substance. Alternatively, when a labeled secondary antibody against the detecting antibody is used, the measured value of ApoA1 can be obtained in the same manner.

In particular embodiments, B/F (Bound/Free) separation for removing unreacted free components which do not form the complex may be performed between the step of forming the complex and the step of detecting the complex. The phrase "unreacted free components" refers to components which do not constitute the complex. Examples of the unreacted free components include the capturing antibody and the detecting antibody both of which have not bound to ApoA1. The means for the B/F separation is not particularly limited. When the solid phase is particles, the B/F separation can be performed by collecting only the solid phase which has captured the complex by centrifugal separation. When the solid phase is a container such as a microplate or a microtube, the B/F separation can be performed by removing the liquid containing unreacted free components. When the solid phase is magnetic particles, the B/F separation can be performed by sucking and removing the liquid containing unreacted free components with a nozzle in a state where the magnetic particles are magnetically bound with a magnet. After the unreacted free components are removed, the solid phase having captured the complex may be washed with an appropriate aqueous medium such as PBS.

In the present description, the phrase "detecting a signal" includes detecting the presence or absence of a signal qualitatively, quantitating a signal intensity and detecting a signal intensity semi-quantitatively. The semi-quantitative detection refers to indicating the signal intensity stepwise. For example, in the semi-quantitative detection, the signal intensity is indicated as "no signal," "weak," "moderate," "strong," and the like. In particular embodiments, it is preferable to detect the signal intensity quantitatively or semi-quantitatively.

The label substance is not particularly limited as long as a detectable signal is generated. For example, the label substance may be a substance which itself generates a signal (hereinafter also referred to as "the signal-generating substance"). The labeled substance may also be a substance which generates a signal by catalyzing a reaction with another substance. Examples of the signal-generating substance include a fluorescent substance and a radioisotope. Examples of the substance which generates a detectable signal by catalyzing a reaction with another substance include an enzyme. Examples of the enzyme include alkaline phosphatase, peroxidase, $\beta$-galactosidase, and luciferase. Examples of the fluorescent substance include fluorescent dyes such as fluorescein isothiocyanate (FITC), rhodamine and Alexa Fluor (registered trademark), and fluorescent proteins such as GFP. Examples of the radioisotope include $^{125}$I, $^{14}$C, and $^{32}$P. Among the above, an enzyme is preferable as the label substance, and alkaline phosphatase and peroxidase are particularly preferable.

The method for detecting a signal itself is known in the art. In the methods provided herein, the measuring method may be appropriately selected depending on the kind of the signal derived from the above-described label substance. For example, when the label substance is an enzyme, the detection can be performed by measuring a signal such as a light and a color generated by reacting the enzyme with a substrate of the enzyme by using a known apparatus such as a spectrophotometer.

A substrate of an enzyme can be appropriately selected from known substrates depending on the kind of the enzyme. For example, when an alkaline phosphatase is used as the enzyme, examples of the substrate include chemiluminescent substrates such as CDP-Star (registered trademark) (4-chloro-3-(methoxyspiro[1, 2-dioxetane-3,2'-(5'-chloro)tricyclo[3.3.1.13,7]decan]-4-yl)phenylphosphoric acid disodium salt) and CSPD (registered trademark) (3-(4-methoxyspiro[1,2-dioxetane-3,2-(5'-chloro)tricyclo[3.3.1.13,7]decan]-4-yl)phenylp hosphoric acid disodium salt) as well as chromogenic substrates such as 5-bromo-4-chloro-3-indolyl phosphate (BCIP), 5-bromo-6-chloro-indolyl phosphate disodium salt and p-nitrophenyl phosphate. When peroxidase is used as the enzyme, examples of the substrate include chemiluminescent substrates such as luminol and derivatives thereof as well as chromogenic substrates such as 2,2'-azino-bis(3-ethylbenzothiazoline-6-sulfonic acid ammonium salt) (ABTS), 1,2-phenylenediamine (OPD) and 3,3',5,5'-tetramethylbenzidine (TMB).

When the label substance is a radioisotope, a radiation as the signal can be measured by using a known apparatus such as a scintillation counter. When the label substance is a fluorescent substance, a fluorescence as the signal can be measured by using a known apparatus such as a fluorescence microplate reader. An excitation wavelength and a fluorescent wavelength can be appropriately determined depending on the kind of the fluorescent substance used.

The result of the signal detection may be used as the measured value of ApoA1. For example, when the signal intensity is detected in a quantitative manner, a measured value of the signal intensity itself or a value obtained from the measured value can be used as the measured value of ApoA1. Examples of the value obtained from the measured value of the signal intensity include a value obtained by subtracting the measured value of a negative control sample or a value of a background from the measured value of ApoA1. The negative control sample can be appropriately selected. Examples of the negative control sample include urine obtained from a healthy subject.

In particular embodiments, a calibration curve which shows the relationship between the concentration of ApoA1 and the measured value of ApoA1 may be created by obtaining measured values of ApoA1 of a plurality of reference samples of which the concentration of ApoA1 is known. The value of the concentration of ApoA1 in urine can be obtained by fitting the measured value of ApoA1 obtained from a urine sample to the calibration curve. It is preferable that the obtained value of the concentration of ApoA1 in urine be corrected by the value of the concentration of creatinine in urine of the subject in order to prevent an error due to the urine volume. Specifically, it is preferable that the value of the concentration of ApoA1 in urine be converted into a concentration of ApoA1 per gram of creatinine ($\mu$g/gCr). By this correction, the values of the concentration of ApoA1 in urine can be compared among subjects.

In particular embodiments, the concentration of ApoA1 in urine may be measured according to a sandwich ELISA method by using a capturing antibody fixed on magnetic particles and a detecting antibody labeled with a label substance. In such a case, the measurement may be performed by using a commercially available fully automatic immunoassay device such as an HISCL series (manufactured by SYSMEX CORPORATION).

Next, in the method according to the present invention, whether a risk of progression to nephropathy in a subject who is a diabetes patient at pre-nephropathy is high or low is determined based on the value of the concentration of ApoA1 in a urine sample of said patient. Specifically, when the value of the concentration of ApoA1 in urine is equal to or higher than a predetermined threshold value, it can be determined that the subject is at a high risk of progression to nephropathy. When the value of the concentration of ApoA1 in urine is lower than the predetermined threshold value, it can be determined that the subject is at a low risk of progression to nephropathy.

When it is determined by the method according to the present invention that a subject is at a high risk of progression to diabetic nephropathy, it may be predicted that the disease stage progresses to incipient nephropathy (Stage 2) or a later stage within a predetermined period of time in the cases where the subject does not receive a treatment. When it is determined that a subject is at a low risk of progression to diabetic nephropathy, it may be predicted that the disease stage does not progress to incipient nephropathy (Stage 2) or a later stage within a predetermined period of time even if the subject does not receive a treatment. Examples of the predetermined period of time include 12 months. Preferable examples of the predetermined period of time include 6 months. In such a manner, the method according to the present invention enables providing information which assists the diagnosis of a risk of progression to diabetic nephropathy to a medical doctor and the like. In other words, the methods provide a prognosis for whether or not a patient at the stage of pre-nephropathy will evolve to diabetic nephropathy. In "Evidence-based Practice Guideline for the Treatment for Diabetes in Japan 2013" (The Japan Diabetes Society), it is instructed that rigorous glycemic control and blood pressure control should be performed in order to prevent the development of nephropathy. It is instructed that the treatment should be performed by administration of an angiotensin-converting enzyme (ACE) inhibitor or an angiotensin II receptor antagonist when the stage progresses to incipient nephropathy which shows microalbuminuria. When it is diagnosed that a diabetes patient at the stage of pre-nephropathy is at a high risk of progression according to the present method, the progression of nephropathy in the diabetes patient can be prevented by administering an ACE inhibitor or an angiotensin II receptor antagonist at an early stage.

The above-described predetermined threshold value is not particularly limited. The predetermined threshold value can be appropriately set. For example, the predetermined threshold value may be set empirically by accumulating data regarding kidney functions including the values of the concentration of ApoA1 in urine of diabetes patients who are at pre-nephropathy. Alternatively, the predetermined threshold value may be set as follows. Firstly, urine is collected from a plurality of diabetes patients who are at pre-nephropathy and the concentration of ApoA1 in urine is measured. After the passage of a given period of time from collecting said urine, a test of the kidney functions of these patients is performed such that disease stages of diabetic nephropathy are confirmed. The data of the measured concentration of ApoA1 in urine are classified into data of a progression group of patients in which diabetic nephropathy has progressed to incipient nephropathy (Stage 2) or a later stage and data of a non-progression group of patients in which diabetic nephropathy has not progressed. Then, the value which is capable of distinguishing most accurately the concentration of ApoA1 in urine of the progression group of patients in which diabetic nephropathy has progressed from the concentration of ApoA1 in urine of the non-progression group of patients in which diabetic nephropathy has not progressed is obtained. The obtained value is used as the predetermined threshold value. It is preferable to consider the sensitivity, specificity, positive predictive value, negative predictive value and the like in determining the threshold value.

A reagent kit used for the above-described diagnosis method of the risk of progression to nephropathy is also included in the scope of the present disclosure. In other words, the present disclosure provides a reagent kit for a diagnosis of a risk of progression to nephropathy in a diabetes patient who is at pre-nephropathy, the reagent kit including: a first capturing body which specifically binds to ApoA1 for capturing ApoA1, a second capturing body which specifically binds to ApoA1 for detecting ApoA1, and a label substance (hereinafter also referred to as "the reagent kit"). When the label substance is an enzyme, the reagent kit according to particular embodiments may further include a substrate of the enzyme.

In the kits of the present invention, the form of each of the first capturing body, the second capturing body, the label substance and the substrate is not particularly limited. The form may be a solid (for example, a powder, a crystal, or a lyophilized preparation) or may be a liquid (for example, a solution, a suspension, or an emulsion). In particular embodiments, it is preferable that each of the first capturing body, the second capturing body, the label substance and the substrate be stored in a separate container or packaged individually. The details of the urine sample, the first capturing body, the second capturing body, the label substance and the substrate are the same as those described in the above-described diagnosis method. In particular embodiments, it is preferable that both the first capturing body and the second capturing body be an anti-ApoAI antibody. When both the first capturing body and the second capturing body are a monoclonal antibody, it is preferable that the epitope for the first capturing body and that for the second capturing body be different from each other.

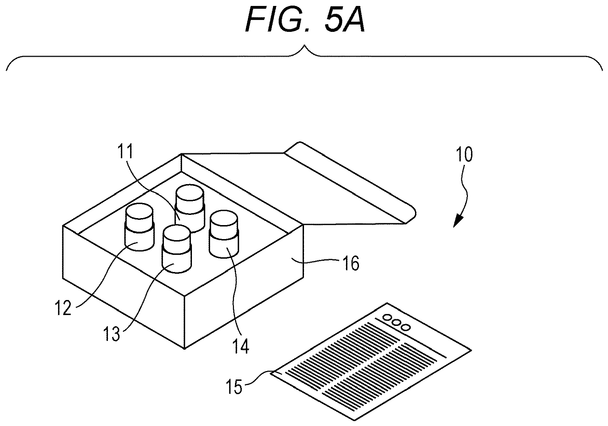

In particular embodiments, containers which accommodate the above-described various reagents may be packaged in a box to be provided to a user. A package insert of the reagent kit may be packaged together in the box. It is preferable that, for example, the constituents of the reagent kit and the protocol for measuring the concentration of ApoA1 in urine be described in the package insert. Fig. 5A shows an example of the appearance of the reagent kit according to a particular embodiment of the invention. In the drawing, reference numeral 10 indicates a reagent kit, 11 indicates a first container which accommodates a first capturing body, 12 indicates a second container which accommodates a second capturing body, 13 indicates a third container which accommodates an enzyme as a label substance, 14 indicates a fourth container which accommodates a substrate of the enzyme, 15 indicates a package insert and 16 indicates a packaging box.

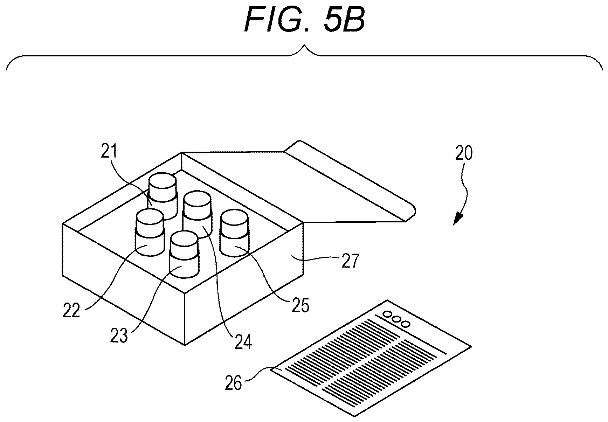

The reagent kit according to the present invention may further include a pretreatment liquid for a pretreatment on urine collected from a subject. The above-described buffer solution is preferable as the pretreatment liquid. The pretreatment liquid may contain a detergent for solubilization of cells which have incorporated ApoA1. The details of the buffer solution and the detergent are the same as those described in the description of the diagnosis method. In particular embodiments, it is preferable that each of the first capturing body, the second capturing body, the label substance, the substrate and the pretreatment liquid be stored in a separate container or packaged individually. Fig. 5B shows an example of the appearance of the reagent kit which further includes the pretreatment liquid. The details of the pretreatment liquid are the same as those described in the description of the diagnosis method. In the drawing, reference numeral 20 indicates a reagent kit, 21 indicates a first container which accommodates a first capturing body, 22 indicates a second container which accommodates a second capturing body, 23 indicates a third container which accommodates an enzyme as a label substance, 24 indicates a fourth container which accommodates a substrate of the enzyme, 25 indicates a fifth container which accommodates a pretreatment liquid, 26 indicates a package insert and 27 indicates a packaging box.

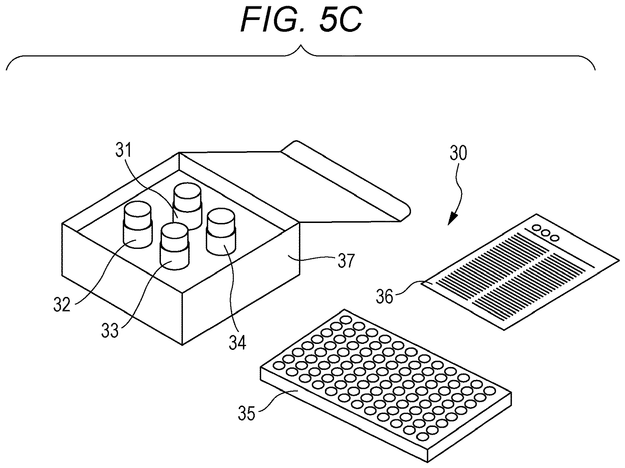

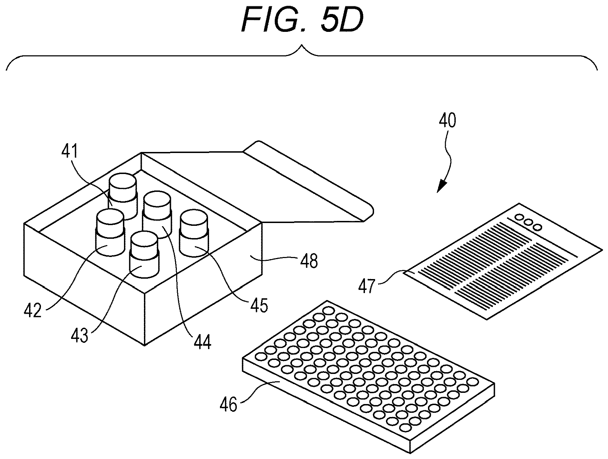

The reagent kit according to particular embodiments may further include a solid phase for fixing the first capturing body. In such a case, it is preferable that each of the first capturing body, the second capturing body, the label substance, the substrate and the solid phase be stored in a separate container or packaged individually. Fig. 5C shows an example of the appearance of the reagent kit which further includes the solid phase. The details of the solid phase are the same as those described in the description of the diagnosis method. In the drawing, reference numeral 30 indicates a reagent kit, 31 indicates a first container which accommodates a first capturing body, 32 indicates a second container which accommodates a second capturing body, 33 indicates a third container which accommodates an enzyme as a label substance, 34 indicates a fourth container which accommodates a substrate of the enzyme, 35 indicates a solid phase (a 96-well microplate), 36 indicates a package insert and 37 indicates a packaging box. Fig. 5D shows an example of the appearance of a reagent kit according to a particular embodiment which further includes the pretreatment liquid. In the drawing, reference numeral 40 indicates a reagent kit, 41 indicates a first container which accommodates a first capturing body, 42 indicates a second container which accommodates a second capturing body, 43 indicates a third container which accommodates an enzyme as a label substance, 44 indicates a fourth container which accommodates a substrate of the enzyme, 45 indicates a fifth container which accommodates a pretreatment liquid, 46 indicates a solid phase (a 96-well microplate), 47 indicates a package insert and 48 indicates a packaging box.

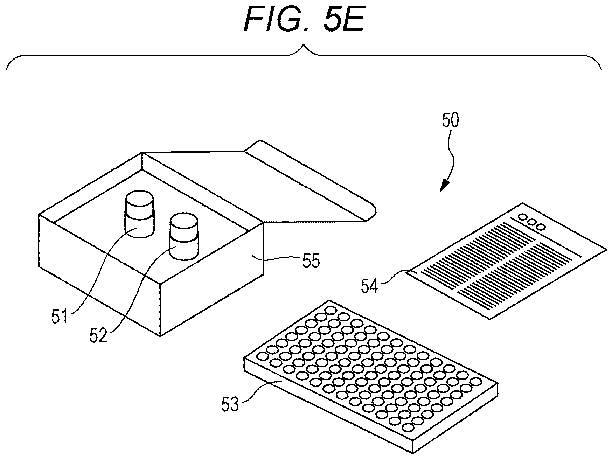

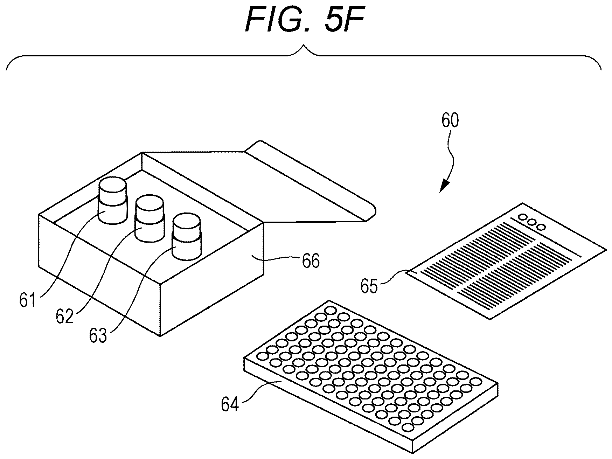

In a further embodiment, the first capturing body may be fixed to the solid phase beforehand. The label substance may be bound to the second capturing body beforehand. In such a case, the reagent kit includes the solid phase which fixes the first capturing body, the second capturing body which binds to the label substance and the substrate. Fig. 5E shows an example of the appearance of the reagent kit according to a particular embodiment. In the drawing, reference numeral 50 indicates a reagent kit, 51 indicates a first container which accommodates a second capturing body which binds to an enzyme as a label substance, 52 indicates a second container which accommodates a substrate of the enzyme, 53 indicates a solid phase (a 96-well microplate) which fixes a first capturing body, 54 indicates a package insert and 55 indicates a packaging box. Fig. 5F shows an example of the appearance of the reagent kit further including the pretreatment liquid according to a particular embodiment. In the drawing, reference numeral 60 indicates a reagent kit, 61 indicates a first container which accommodates a second capturing body which binds to an enzyme as a label substance, 62 indicates a second container which accommodates a substrate of the enzyme, 63 indicates a third container which accommodates a pretreatment liquid, 64 indicates a solid phase (a 96-well microplate) which fixes a first capturing body, 65 indicates a package insert and 66 indicates a packaging box.

Additionally or alternatively, the reagent kit according to the present invention may further include a washing solution for the solid phase, an enzymatic reaction terminator, a calibrator and the like, appropriately in addition to the above-described various reagents.

The use of the above-described reagents for manufacturing the reagent kit for the diagnosis of the risk of progression to nephropathy is also included in the scope of the present disclosure. In other words, the present disclosure also relates to the use of the first capturing body which specifically binds to ApoA1 for capturing ApoA1, the second capturing body which specifically binds to ApoA1 for detecting ApoA1 and the label substance for manufacturing the reagent kit for the diagnosis of the risk of progression to nephropathy.

The present invention is described herein below in more detail by referring to examples. However, the present invention is not limited to the examples.

EXAMPLES

Example 1

Blood and urine were collected from subjects who were diabetes patients at pre-nephropathy (Stage 1). After 6 months, the subjects were divided into a progression group and a non-progression group of nephropathy. The concentrations of ApoA1 and ApoB in blood and urine of subjects of each of the groups were compared with each other. ApoB is known as a biomarker of a renal disease.

(1) Sampling from subjects

Blood and urine were collected as samples from diabetes patients (n = 14) each having a urine albumin-to-Cr ratio of lower than 30 mg/gCr and a GFR category of equal to or higher than 30 mL/min/1.73 m$^2$.

(2) Measurements of biomarkers

(2.1) Measurements of the concentrations of ApoA1, ApoB and creatinine in serum

The concentrations of ApoA1 and ApoB in serum from blood of each of the subjects obtained in step (1) were measured by using ApoA-I Auto-N "Daiichi" and ApoB Auto-N "Daiichi" (both of them were from SEKISUI MEDICAL CO., LTD.). Specific procedures were in accordance with a package insert of each of the kits. The concentration of serum creatinine (sCR) from blood of each of the subjects was measured by using L-system-CRE (SYSMEX CORPORATION) and CRE Standard Solution (SYSMEX CORPORATION). Specific procedures were in accordance with package inserts of the reagents.

(2.2) Measurements of the concentrations of ApoA1, ApoB and creatinine in urine

Equal amounts of the urine obtained from each of the subjects obtained in step (1) and Triton-X100/PBS included in the kit described below were mixed, and thereafter, the mixture was diluted 25-fold with a sample dilution solution. The concentrations of ApoA1 and ApoB in urine were measured from the diluted urine by using Human Apolipoprotein A1 ELISA Pro Kit (MABTECH AB) and Human Apolipoprotein B ELISA Pro Kit (MABTECH AB). Specific procedures were in accordance with a package insert of each of the kits, except that the diluted urine was used instead of the serum. The concentration of creatinine in urine from urine of each of the subjects was measured by using L-system-CRE (SYSMEX CORPORATION) and CRE Standard Solution (SYSMEX CORPORATION). Specific procedures were in accordance with package inserts of the reagents. In order to correct an error due to the urine volume, each of the measured concentrations of ApoA1 and ApoB in urine was converted to a concentration per gram of creatinine ($\mu$g/gCr) by using the concentration of creatinine in urine.

(3) Classification of subjects

Blood was collected from each of the subjects 6 months after the sampling step (1). The concentration of sCR was measured from the obtained blood by using L-system-CRE (SYSMEX CORPORATION) and CRE Standard Solution (SYSMEX CORPORATION). From each of the measured value of sCR at the time of sampling and the measured value of sCR 6 months after sampling, the estimated glomerular filtration rate (eGFR) was calculated according to the formula below. In the formula, "Age" represents the age of the subject.

$$\text{eGFR (mL/min/1.73 m}^2) = 194 \times \text{sCr}^{-1.094} \times \text{Age}^{-0.287} \text{ (when the subject is female, eGFR}$$

$$= 194 \times \text{sCr}^{-0.739} \times \text{Age}^{-0.287})$$

From the eGFR at the time of sampling and the eGFR 6 months after sampling, the rate of change (%) in eGFR per year was calculated according to the formula below. The obtained value of the rate of change was considered to be the progression index (%) of nephropathy. A subject having a progression index of equal to or higher than 0 was classified into a non-progression group of nephropathy (hereinafter also referred to as "the non-progression group," n = 7) and a subject having a progression index of lower than 0 was classified into a progression group of nephropathy (hereinafter also referred to as "the progression group," n = 7).

$$\text{Rate of change (\%)} = [(\text{eGFR 6 months after sampling - eGFR at the time of}$$

$$\text{sampling})/(\text{eGFR at the time of sampling})] \times \text{interval in days between measurements}$$

$$\text{(days)} \times 100/365$$

(4) Results

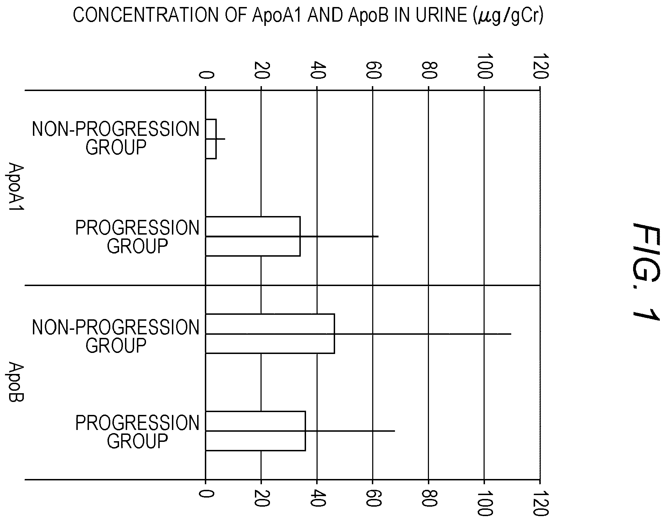

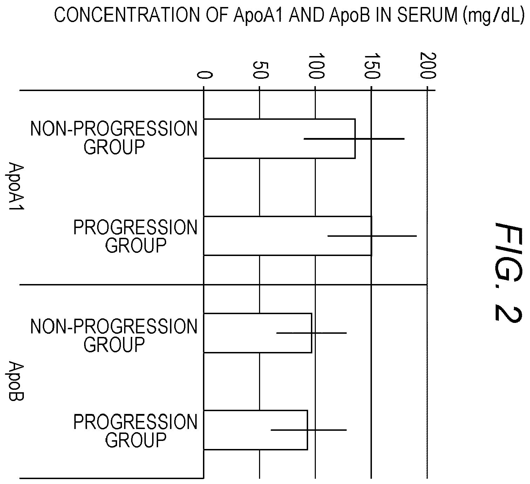

Fig. 1 shows the concentrations of ApoA1 and ApoB in urine of the non-progression group and the progression group. As shown in Fig. 1, the concentration of ApoA1 in urine of the progression group was significantly higher than the concentration of ApoA1 in urine of the non-progression group. On the other hand, no statistically significant difference was recognized between the progression group and the non-progression group with regard to the concentration of ApoB in urine. Fig. 2 shows the concentrations of ApoA1 and ApoB in serum of the non-progression group and the progression group. As shown in Fig. 2, no statistically significant difference was recognized between the progression group and the non-progression group with regard to the concentrations of ApoA1 and ApoB in serum. No statistically significant difference was recognized between the progression group and the non-progression group with regard to the serum ApoB/ApoA1 ratios, either. Patel ML. et al. (Clinical Correlation between ApoB/Apo-A1 Ratio in Type 2 Diabetic Nephropathy-A Tertiary Centre Experience, Int. J. Sci. Res. Pub, Vol. 2, Issue 9, 1 - 8 (2012): Non-Patent Document 2) describe that the serum ApoB/ApoA1 ratio may be an early indicator of diabetic nephropathy. However, from the results of the present example, it was shown that the serum ApoB/ApoA1 ratio cannot be utilized for determining the risk of progression to nephropathy in a diabetes patient who is at pre-nephropathy (Stage 1).

Based on the above, it was suggested that the risk of progression to nephropathy in a diabetes patient who is at pre-nephropathy (Stage 1) can be predicted based on the concentration of ApoA1 in urine.

Example 2

With regard to subjects who were diabetes patients at incipient nephropathy (Stage 2) and those at overt nephropathy (Stage 3), whether the risk of progression of nephropathy can be predicted based the concentration of ApoA1 in urine or not was also studied.

(1) Sampling from subjects

Blood and urine were collected as samples from diabetes patients each having a urine albumin-to-Cr ratio of equal to or higher than 30 mg/gCr and lower than 300 mg/gCr and a GFR category of equal to or higher than 30 mL/min/1.73 m$^2$ (Stage 2, n = 6) as well as diabetes patients each having a urine albumin-to-Cr ratio of equal to or higher than 300 mg/gCr and a GFR category of equal to or higher than 30 mL/min/1.73 m$^2$ (Stage 3, n = 8).

(2) Measurements of biomarkers

The concentration of ApoA1 in urine as well as the concentration of creatinine in serum and urine were measured in the same manner as in Example 1. The concentration of ApoA1 in urine per gram of creatinine was calculated from the concentration of ApoA1 in urine and the concentration of creatinine in urine.

(3) Classification of subjects and ROC analyses

Blood was collected from each of the subjects 6 months after the sampling step (1) and the concentration of sCR was measured. The progression index (%) of nephropathy was calculated from the concentration of sCR at the time of sampling and 6 months after the sampling in the same manner as in Example 1. The subjects were classified into a progression group and a non-progression group of nephropathy. With regard to each of the subjects at Stage 2 and those at Stage 3, the accuracy of determination was examined by an ROC (Receiver Operating Characteristic) curve analysis about a case where the risk of progression of nephropathy was determined based on the concentration of ApoA1 in urine. With regard to the subjects at pre-nephropathy of Example 1, an ROC analysis was performed for the purpose of comparison in the same manner.

(4) Results

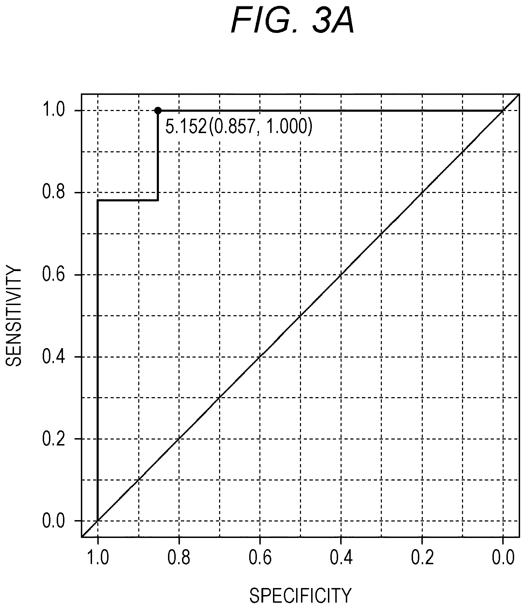

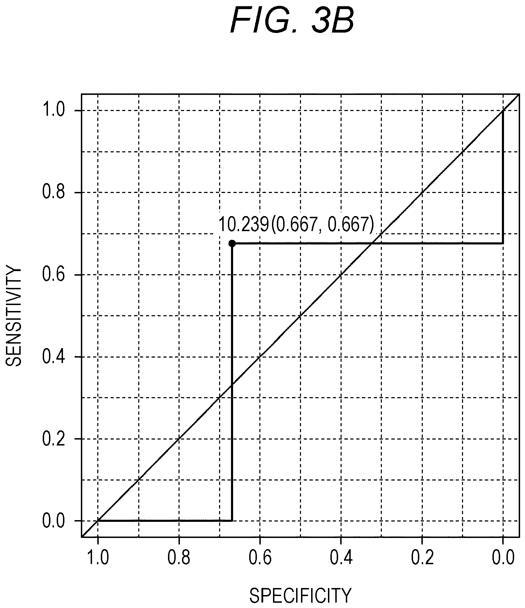

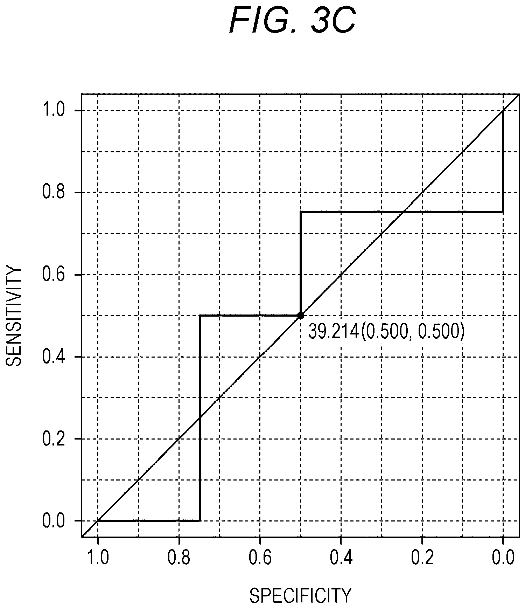

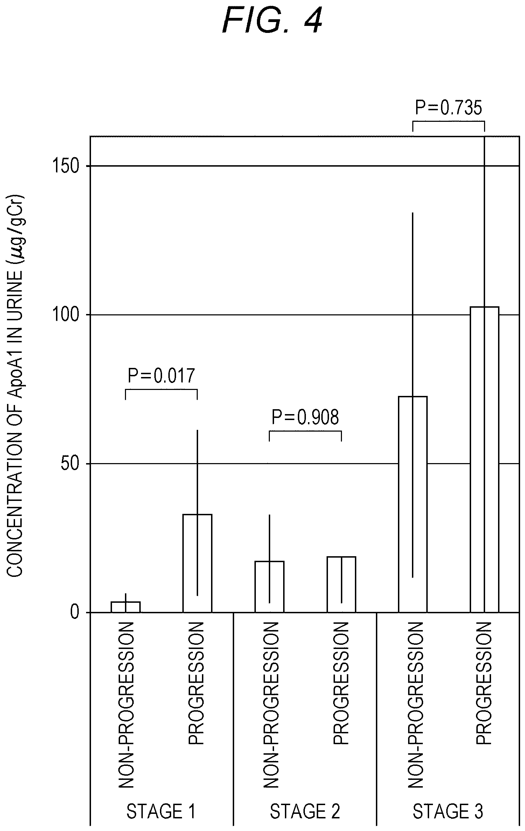

The result of each of the ROC analyses was evaluated by the value of AUC (Area Under the Curve). Each of Fig. 3A (Stage 1), Fig. 3B (Stage 2) and Fig. 3C (Stage 3) shows the ROC curve obtained by using subjects at each of the disease stages. Table 2 shows the AUC of each of the ROC curves. Fig. 4 shows the concentration of ApoA1 in urine of the subjects of the progression group and the non-progression group at each of the disease stages.

[Table 2]

| Disease stage | AUC |
| --- | --- |
| Pre-nephropathy (Stage 1) | 0.968 |
| Incipient nephropathy (Stage 2) | 0.444 |
| Overt nephropathy (Stage 3) | 0.500 |

As shown in Fig. 3B, Fig. 3C and Fig. 4, with regard to the diabetes patients at incipient nephropathy (Stage 2) and overt nephropathy (Stage 3), there was no significant difference in the results of determination of the risk of progression of nephropathy based on the concentration of ApoA1 in urine. On the other hand, as shown in Fig. 3A and Fig. 4, with regard to the diabetes patients at pre-nephropathy (Stage 1), there was a significant difference in the results of determination of the risk of progression to nephropathy based on the concentration of ApoA1 in urine.

As described above, Gomo ZA., et al. (High-Density Lipoprotein Apolipoproteins in Urine: II. Enzyme-Linked Immunoassay of Apolipoprotein A-I, Clinical Chemistry, Vol. 34, No. 9, 1781-1786 (1988): Non-Patent Document 1) report that there was a difference of thousandfold or more in the concentration of ApoA1 in urine between patients with renal dysfunction and healthy subjects. Based on this result, Gomo ZA., et al. suggest a perspective that the measurement of ApoA1 in urine makes it possible that patients with asymptomatic nephropathy and healthy subjects are discriminated from each other. It can be considered based on the discussion by Gomo ZA., et al. that ApoA1 in urine, which is a biomarker normally used for discriminating patients with nephropathy at Stage 2 or a later stage from healthy subjects, can also be utilized as a marker for discriminating patients at pre-nephropathy from healthy subjects. However, in the present example, the results contrary to such a teaching by Gomo ZA., et al. were obtained. The present inventors surprisingly found that ApoA1 in urine, which is a biomarker which cannot be used for determining the risk of progression in diabetes patients with nephropathy at Stage 2 or a later stage, is an extremely useful marker for determining the risk of progression in diabetes patients at pre-nephropathy.

Example 3

Whether the risk of progression to nephropathy in diabetes patients at pre-nephropathy (Stage 1) can also be determined based on the urinary concentration of biomarkers for renal diseases other than ApoA1 in urine or not was studied.

(1) Sampling from subjects

Blood and urine were collected as samples from diabetes patients each having a urine albumin-to-Cr ratio of lower than 30 mg/gCr as well as a GFR category of equal to or higher than 30 mL/min/1.73 m$^2$ (n = 14).

(2) Measurements of biomarkers

The urinary concentrations of α1-microglobulin (α1M), Kidney injury molecule-1 (KIM-1), N-acetylglucosaminidase (NAG), type IV collagen, urine albumin (uALB), urine protein (uTP), lipocalin-2 (NGAL) and L type fatty acid binding protein (LFABP) were measured as the biomarkers in urine. The concentration of α1M was measured by using LZ Test "EIKEN"α-1M (Eiken Chemical Co., Ltd.) and LZ-α1-M Standard "EIKEN" for measuring α-1M in urine (Eiken Chemical Co., Ltd.) in accordance with package inserts. The concentration of KIM-1 was measured by using KIM-1 (human) Elisa Kit (Enzo life sciences Inc.) in accordance with a package insert. The concentration of NAG was measured by using L-Type Wako NAG (Wako Pure Chemical Industries, Ltd.) and NAG Calibrator (Wako Pure Chemical Industries, Ltd.) in accordance with package inserts. The concentration of type IV collagen was measured by using Panauria (registered trademark) uIV-C "Plate" (Kyowa Pharma Chemical Co., Ltd.) in accordance with a package insert. The concentration of uALB was measured by using LZ Test "EIKEN" U-ALB (Eiken Chemical Co., Ltd.) and LZ-U-ALB Calibrator "EIKEN"(Eiken Chemical Co., Ltd.) in accordance with package inserts. The concentration of uTP was measured by using Micro TP-AR (Wako Pure Chemical Industries, Ltd.) and Protein Standard Solution (Protein 100 mg/dL, Wako Pure Chemical Industries, Ltd.) in accordance with package inserts. The concentration of NGAL was measured by using NGAL (human) Elisa Kit (Enzo life sciences Inc.) in accordance with a package insert except that urine was diluted 50-fold with a sample dilution solution included in the kit. The concentration of LFABP was measured by using Nordia (registered trademark) L-FABP (SEKISUI MEDICAL CO., LTD.) and L-FABP Calibrator (SEKISUI MEDICAL CO., LTD.) in accordance with package inserts. For the purpose of comparison, the concentrations of ApoA1 in urine and ApoB in serum were measured in the same manner as in Example 1. The concentration of creatinine in urine was measured by using L-system-CRE (SYSMEX CORPORATION) and CRE Standard Solution (SYSMEX CORPORATION) in accordance with package inserts. In order to correct an error due to the urine volume, each of the measured concentrations of the biomarkers was converted to a concentration per gram of creatinine (μg/gCr) by using the concentration of creatinine in urine. In addition, the concentration of sCr was measured from blood of each of the subjects in the same manner as in Example 1.

(3) Classification of subjects

Blood was collected from each of the subjects 6 months after the sampling step (1) and the concentration of sCR was measured. The progression index (%) of nephropathy was calculated from the concentration of sCR at the time of sampling and 6 months after the sampling in the same manner as in Example 1. The subjects were classified into a progression group and a non-progression group of nephropathy.

(4) Statistical analyses

With regard to the concentration of each of the biomarkers, whether there is a statistically significant difference between the progression group and the non-progression group or not was examined. Specifically, with regard to each of the biomarkers, the normality of data was confirmed and whether the variance of the progression group and that of the non-progression group are the same or not was confirmed. Then, whether the difference in the mean values of the progression group and the non-progression group is the same or not was tested. With regard to each of the biomarkers, the accuracy of determination was examined by the ROC curve analysis and was evaluated by the value of AUC about a case were whether there was a progression or not was determined. In addition, with regard to each of the biomarkers, whether the measured value of the biomarker in urine enables prediction of the value of the progression index (the value of serum creatinine or the value of eGFR) 6 months after sampling or not was evaluated by the single regression analysis.

(5) Results

Table 3 shows the results of analyses. In Table 3, when the significance level P-value is lower than 0.05, it can be considered that there is a significant difference. It can be considered that the closer to 1 the value of AUC is, the higher the accuracy of determination is. With regard to L-FABP, all the samples at pre-nephropathy had the measured value of zero (equal to or lower than the detection limit), and therefore, the evaluation could not be performed.

[Table 3]

| Biomarker | Significant difference in mean values between progression group and non-progression group (significance level P-value) | Ratio between mean values (mean value of progression group/mean value of non-progression group) | AUC of ROC curve | Prediction of progression index by single regression analysis (significance level P-value) |
|---|---|---|---|---|
| ApoA1 | 0.017 | 8.4 | 0.968 | 0.016 |
| α1-M | 0.340 | 0.6 | 0.490 | 0.541 |
| KIM-1 | 0.429 | 0.8 | 0.735 | 0.527 |
| NAG | 0.948 | 1.0 | 0.510 | 0.921 |
| Type IV collagen | 0.634 | 1.4 | 0.653 | 0.903 |
| ApoB | 0.690 | 0.8 | 0.592 | 0.330 |
| uALB | 0.178 | 1.5 | 0.765 | 0.782 |
| uTP | 0.975 | 1.0 | 0.582 | 0.856 |
| NGAL | 0.963 | 1.0 | 0.571 | 0.766 |
| L-FABP | - | - | - | - |

As shown in Table 3, there was a difference of eight-fold or more in the mean values of the concentration of ApoA1 in urine between the progression group and the non-progression group, and it was found that there is a statistically significant difference between both the groups. With regard to ApoA1, AUC exceeds 0.9, and the changed level of eGFRs from the time of sampling to 6 months after the sampling could be predicted based on the concentration of ApoA1 in urine. On the other hand, with regard to each of the biomarkers other than ApoA1, there was no statistically significant difference between the progression group and the non-progression group. These biomarkers are known to be urine markers for nephropathy, but among them, there was no marker that is capable of determining the risk of progression to nephropathy in diabetes patients at pre-nephropathy (Stage 1).

**Claims**

1. A method for assisting a diagnosis of a risk of progression to nephropathy, the method comprising the steps of:

    measuring a concentration of apolipoprotein A1 (ApoA1) in urine of a diabetes patient who is at pre-nephropathy; and
    determining that the patient is at a high risk of progression to nephropathy when a value of the concentration of ApoA1 in urine is equal to or higher than a predetermined threshold value or determining that the patient is at a low risk of progression to nephropathy when the value of the concentration of ApoA1 in urine is lower than the predetermined threshold value.

2. The method according to claim 1, wherein the concentration of ApoA1 in urine is measured by using a capturing body which specifically binds to ApoA1.

3. The method according to claim 2, wherein the capturing body which specifically binds to ApoA1 is an anti-ApoAl antibody.

4. The method according to any one of claims 1 to 3, wherein the diabetes patient who is at pre-nephropathy has a urine albumin-to-creatinine ratio of lower than 30 mg/gCr and has an estimated GFR of equal to or higher than 30 mL/min/1.73 m$^2$.

5. The method according to any one of claims 1 to 4, wherein the value of the concentration of ApoA1 in urine is a value corrected by a value of a concentration of creatinine in urine of the patient.

6. The method according to any one of claims 1 to 5, wherein the concentration of ApoA1 is measured by immunoassay in the measuring step.

7. Use of a reagent kit for the method according to any one of claims 1 to 6, wherein the reagent kit comprises:

a first capturing body which specifically binds to ApoA1 for capturing ApoA1;
a second capturing body which specifically binds to ApoA1 for detecting ApoA1; and
a label substance.

8. The use of the reagent kit according to claim 7, wherein the first capturing body is an anti-ApoAl antibody.

9. The use of the reagent kit according to claim 7 or 8, wherein the second capturing body is an anti-ApoAl antibody.

10. The use of the reagent kit according to any of claims 7 to 9, wherein the label substance is at least one selected from the group consisting of fluorescent substance, radioisotope and enzyme.

11. The use of the reagent kit according to any of claims 7 to 10, wherein
the reagent kit further comprises a solid phase, and
the first capturing body is fixed on the solid phase.

12. The use of the reagent kit according to claim 11, wherein the solid phase is a particle, membrane, microplate, microtube, or test tube.

13. The use of the reagent kit according to any of claims 7 to 12, wherein
the first capturing body is an anti-ApoAl antibody and the second capturing body is an anti-ApoAl antibody, and
an epitope of the first capturing body is different from an epitope of the second capturing body.


**Patentansprüche**

1. Verfahren zur Unterstützung der Diagnose eines Risikos für das Fortschreiten zu Nephropathie, umfassend die folgenden Schritte:

Messen einer Konzentration von Apolipoprotein A1 (ApoA1) im Urin eines Diabetespatienten, der sich im Zustand vor Nephropathie befindet; und
Feststellen, dass der Patient ein hohes Risiko für das Fortschreiten zu Nephropathie hat, wenn ein Wert der Konzentration von ApoA1 im Urin gleich oder höher ist als ein festgelegter Schwellenwert, oder Feststellen, dass der Patient ein geringes Risiko für das Fortschreiten zu Nephropathie hat, wenn der Wert der Konzentration von ApoA1 im Urin niedriger ist als der festgelegte Schwellenwert.

2. Verfahren nach Anspruch 1, wobei die Konzentration von ApoA1 im Urin durch Verwendung eines Einfangkörpers gemessen wird, der spezifisch an ApoA1 bindet.

3. Verfahren nach Anspruch 2, wobei der Einfangkörper, der spezifisch an ApoA1 bindet, ein Anti-ApoA1-Antikörper ist.

4. Verfahren nach einem der Ansprüche 1 bis 3, wobei der Diabetespatient, der sich im Zustand vor Nephropathie befindet, ein Albumin-zu-Kreatinin-Verhältnis im Urin von weniger als 30 mg/g Kreatinin aufweist und eine geschätzte GFR von gleich oder mehr als 30 ml/min/1,73 m$^2$ aufweist.

5. Verfahren nach einem der Ansprüche 1 bis 4, wobei der Wert der Konzentration von ApoA1 im Urin ein Wert ist, der durch einen Wert einer Konzentration von Kreatinin im Urin des Patienten korrigiert ist.

6. Verfahren nach einem der Ansprüche 1 bis 5, wobei die Konzentration von ApoA1 in dem Messschritt mittels Immunassay gemessen wird.

7. Verwendung eines Reagenzienkits für das Verfahren nach einem der Ansprüche 1 bis 6, wobei das Reagenzienkit umfasst:

einen ersten Einfangkörper, der spezifisch an ApoA1 bindet, zum Einfangen von ApoA1;
einen zweiten Einfangkörper, der spezifisch an ApoA1 bindet, zum Nachweisen von ApoA1;
und
eine Markierungssubstanz.

8. Verwendung des Reagenzienkits nach Anspruch 7, wobei der erste Einfangkörper ein Anti-ApoA1-Antikörper ist.

9. Verwendung des Reagenzienkits nach Anspruch 7 oder 8, wobei der zweite Einfangkörper ein Anti-ApoA1-Antikörper ist.

10. Verwendung des Reagenzienkits nach einem der Ansprüche 7 oder 9, wobei die Markierungssubstanz wenigstens eine ausgewählt aus der Gruppe ist, die aus einer Fluoreszenzsubstanz, einem Radioisotop und einem Enzym besteht.

11. Verwendung des Reagenzienkits nach einem der Ansprüche 7 bis 10, wobei
das Reagenzienkit ferner eine feste Phase umfasst, und
der erste Einfangkörper auf der festen Phase fixiert ist.

12. Verwendung des Reagenzienkits nach Anspruch 11, wobei die feste Phase ein Partikel, eine Membran, eine Mikroplatte, ein Mikroröhrchen, oder ein Teströhrchen ist.

13. Verwendung des Reagenzienkits nach Anspruch 7 bis 12, wobei
der erste Einfangkörper ein Anti-ApoA1-Antikörper ist und der zweite Einfangkörper ein Anti-ApoA1-Einfangkörper ist, und
ein Epitop des ersten Einfangkörpers von einem Epitop des zweiten Einfangkörpers verschieden ist.


**Revendications**

1. Procédé pour faciliter un diagnostic d'un risque de progression en néphropathie, le procédé comprenant les étapes de :

mesure d'une concentration d'apolipoprotéine A1 (ApoA1) dans l'urine d'un patient diabétique qui est dans un état de prénéphropathie ; et
détermination que le patient présente un risque élevé de progression en néphropathie lorsqu'une valeur de la concentration d'ApoA1 dans l'urine est égale ou supérieure à une valeur de seuil prédéterminée ou détermination que le patient présente un faible risque de progression en néphropathie lorsque la valeur de la concentration d'ApoA1 dans l'urine est inférieure à la valeur de seuil prédéterminée.

2. Procédé selon la revendication 1, dans lequel la concentration d'ApoA1 dans l'urine est mesurée au moyen d'un corps de capture qui se lie spécifiquement à ApoA1.

3. Procédé selon la revendication 2, dans lequel le corps de capture qui se lie spécifiquement à ApoA1 est un anticorps anti-ApoA1.

4. Procédé selon l'une quelconque des revendications 1 à 3, dans lequel le patient diabétique qui est dans un état de prénéphropathie présente un rapport albumine-créatinine dans l'urine inférieur à 30 mg/gCr et présente un DFG estimé égal ou supérieur à 30 mL/min/1,73 m$^2$.

5. Procédé selon l'une quelconque des revendications 1 à 4, dans lequel la valeur de la concentration d'ApoA1 dans l'urine est une valeur corrigée par une valeur d'une concentration de créatinine dans l'urine du patient.

6. Procédé selon l'une quelconque des revendications 1 à 5, dans lequel la concentration d'ApoA1 est mesurée par immunoessai dans l'étape de mesure.

7. Utilisation d'un kit de réactifs pour le procédé selon l'une quelconque des revendications 1 à 6, le kit de réactifs comprenant :

un premier corps de capture qui se lie spécifiquement à ApoA1 pour capturer ApoA1 ; un deuxième corps de capture qui se lie spécifiquement à ApoA1 pour détecter ApoA1 ; et
une substance de marquage.

8.  Utilisation du kit de réactifs selon la revendication 7, dans laquelle le premier corps de capture est un anticorps anti-ApoA1.

9.  Utilisation du kit de réactifs selon la revendication 7 ou 8, dans laquelle le deuxième corps de capture est un anticorps anti-ApoA1.

10. Utilisation du kit de réactifs selon l'une quelconque des revendications 7 à 9, dans laquelle la substance de marquage est au moins l'un choisi dans le groupe constitué d'une substance fluorescente, d'un radioisotope et d'une enzyme.

11. Utilisation du kit de réactifs selon l'une quelconque des revendications 7 à 10, dans laquelle
le kit de réactifs comprend en outre une phase solide, et le premier corps de capture est fixé sur la phase solide.

12. Utilisation du kit de réactifs selon la revendication 11, dans laquelle la phase solide est une particule, une membrane, une microplaque, un microtube ou un tube à essai.

13. Utilisation du kit de réactifs selon l'une quelconque des revendications 7 à 12, dans laquelle
le premier corps de capture est un anticorps anti-ApoA1 et le deuxième corps de capture est un anticorps anti-ApoA1, et un épitope du premier corps de capture est différent d'un épitope du deuxième corps de capture.

## FIG. 1

# FIG. 2

## FIG. 3A

# FIG. 3B

## FIG. 3C

# FIG. 4

# FIG. 5A

# FIG. 5B

# FIG. 5C

# FIG. 5D

# FIG. 5E

# FIG. 5F

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20030017523 A
- EP 1300683 A1
- EP 0303229 A
- JP SHO6382350 B
- JP HEI1254868 B

### Non-patent literature cited in the description

- **GOMO ZA et al.** High-Density Lipoprotein Apolipoproteins in Urine: II. Enzyme-Linked Immunoassay of Apolipoprotein A-I. *Clinical Chemistry,* 1988, vol. 34 (9), 1781-1786
- **PATEL ML et al.** Clinical Correlation between ApoB/Apo-A1 Ratio in Type 2 Diabetic Nephropathy-A Tertiary Centre Experience. *Int. J. Sci. Res. Pub,* 2012, vol. 2 (9), 1-8
- **CALANDRA et al.** *Experimental and Molecular Pathology,* 1983, vol. 39, 282-299
- **BRITES et al.** *Diabetes Research and Clinical Practice,* 2007, vol. 75, 35-41
- **GOMO ZA. et al.** High-Density Lipoprotein Apolipoproteins in Urine: II. Enzyme-Linked Immunoassay of Apolipoprotein A-I. *Clinical Chemistry,* 1988, vol. 34 (9), 1781-1786
- Evidence-based Practice Guideline for the Treatment for Diabetes in Japan. The Japan Diabetes Society, 2013
- **GOMO ZA. et al.** High-Density Lipoprotein Apolipoproteins in Urine: II. Enzyme-Linked Immunoassay of Apolipoprotein A-I. *Clinical Chemistry,* 1988, vol. 34 (9), 1781-1786